# EUROPEAN PATENT APPLICATION

(11) **EP 0 548 521 A1**
(43) Date of publication of application: **30.06.1993**
(21) Application number: 92119139.1
(22) Date of filing: 09.11.1992
(51) Int. Cl.: A61B 17/11

(54) **Anastomotic device**

(30) Priority: 26.12.1991 US 814023
(71) Applicant: AMERICAN CYANAMID COMPANY, Wayne, NJ 07470-8426 (US)
(72) Inventor: Machado, Fidelis Rosario Christie, London W13 8JE (GB)
(74) Representative: Wächtershäuser, Günter, Prof. Dr.

(57) **Abstract**

An anastomotic device is described for receiving the free ends of two anatomic tubular structures (20,22). The device has a pair of ring members for securement to the free end of each of the tubular structures to be anastomosed. The ring members have annular connecting structures which mate with each other to connect the ring members. The annular connecting structures enable the securement of the ring members in an adjustable fixed relationship at predetermined distances from each other.

## Description

This invention relates to an anastomotic device and method and particularly to a surgical clamp and method for using same for anastomosing one hollow or tubular member to another such as, for example, the severed ends of the intestine after surgery.

Present medical techniques for joining the severed ends of the intestine include stitching the severed ends of the vessel together or using stapling instruments, all of which have some inherent disadvantages. Technical difficulties in utilizing suturing techniques often occur because of the inaccessibility of one or both of the ends to be joined or the time involved for anastomosis. Stapling instruments also have some inherent disadvantages because of the technical problems associated with the position of a severed tubular member within the patient.

It is desirable that a nonpermanent connector or junction device be used to join the tubular member ends in anastomotic surgery since a permanent connector will tend to prevent the changes in diameter which are necessary for the proper functioning of the intestine. Any foreign substance used in anastomotic surgery ideally should disintegrate in a relatively short period of time once healing of the vessel ends is established.

The present invention has been developed to meet the current requirements of anastomotic surgery and to provide a safe, easy-to-use anastomotic device. It is a disintegratable anastomotic article which has locking features to accommodate abutting ends of tubing within a range of tubing wall thicknesses.

The improved anastomotic device and method of the present invention is characterized by one or more engageable, locking slots carried by mating prongs and a plurality of points carried by separate prongs which cooperatively join the ring members and retain the ring members in one of several preselected relationships with each other after being closed from the open position. The distance between ring members can be varied using the ratcheting mechanism to accommodate different thicknesses of tubular member walls. One device can therefore be used on different thicknesses of tubular member walls. The ring members and affixed prongs are so designed that they consist of a single unitary member that can be injection-molded. Two such molded parts can be put together without difficulty so that an anastomotic device capable of mass production and easy assembly is provided.

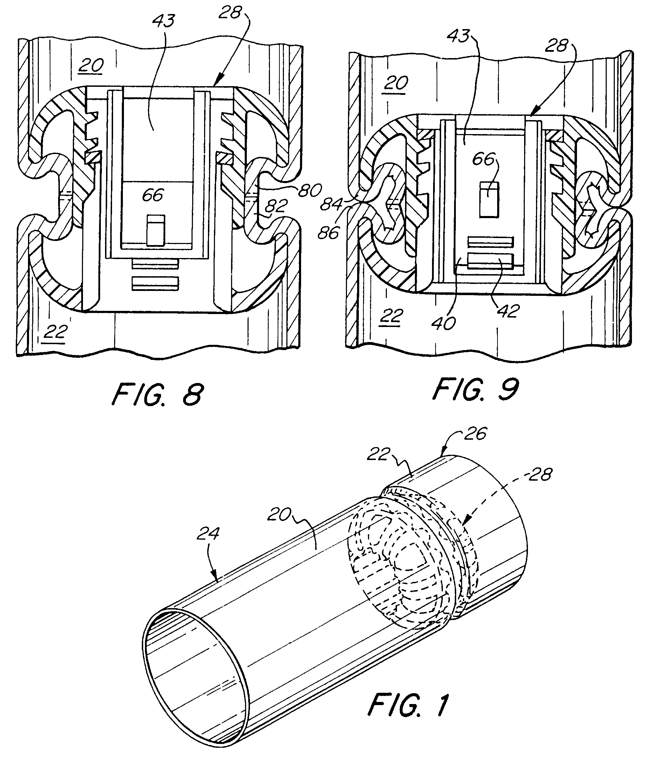

Further details are explained below with the help of examples illustrated in the attached drawing wherein like characters of reference designate like parts throughout the several views and in which:
**FIG. 1** is a perspective view of an anastomotic device made in accordance with the present invention shown in broken lines in the closed position and positioned to join the free ends of a tubular member in a contiguous relationship with each other.
**FIG. 2** is a bottom view of a single unitary member of a first embodiment of the anastomotic device having two such members comprising the present invention and shown in **FIG. 1**;
**FIG. 3** is a side elevational and sectional view of a first embodiment of the anastomotic device according to the present invention including two unitary members like that shown in **FIG. 2** and shown in solid lines in an engaged but unlocked position and in broken lines in an engaged and locked position;
**FIG. 4** is a side elevational and sectional view of the single unitary member of **FIG. 2** having a single ring member and an associated mating prong with locking notches and separate prong with pawls formed therein;
**FIG. 5** is a top view of the single unitary member shown in **FIG. 4**;
**FIG. 6** is a fragmentary and sectional view of the locking notches carried by the mating prong shown in **FIG. 4**;
**FIG. 7** is an isolated side elevational and sectional view of the single unitary member shown in **FIG. 4** showing an engaging pawl carried by the separate prong;
**FIG. 8** is a sectional view of a first embodiment of the anastomotic device comprising the present invention joining the free ends of a tubular member with the device in the open or unlocked position;
**FIG. 9** is a sectional view of a first embodiment of the anastomotic device comprising the present invention joining the free ends of a tubular member with the device in the closed position;
**FIG. 10** is a plain view of a single unitary member of a second embodiment of the anastomotic device comprising the present invention;
**FIG. 11** is a side view of a second embodiment of the anastomotic device according to the present invention shown in solid lines in an engaged but unlocked position and in broken lines in an engaged and locked position;
**FIG. 12** is a side elevational view of the single unitary member of **FIG. 2** having a single ring member and an associated mating prong with locking notches and a separate prong with engaging pawls formed therein;
**FIG. 13** is a top view of the single unitary member shown in **FIG. 12**;
**FIG. 14** is a fragmentary sectional view of the single unitary member shown in **FIG. 13** taken along line **14-14**;
**FIG. 15** is an isolated side elevational view of the single unitary member shown in **FIG. 13** taken along line **15-15**;
**FIG. 16** is a sectional view of the second embodiment of the anastomotic device comprising the present invention joining the free ends of a tubular member with the device in the open or unlocked position; and
**FIG. 17** is a sectional view of the second embodiment of the anastomotic device comprising the present invention joining the free ends of the tubular member with the device in the closed position;
Referring now to the drawings and particularly to **FIG. 1**, the free ends **20** and **22** of two tubular tissue members, shown here as cut ends of intestinal tracts and referred to generally as **24** and **26**, have been anastomosed by using the device comprising the present invention shown by broken lines in outline form and designated generally as **28**.

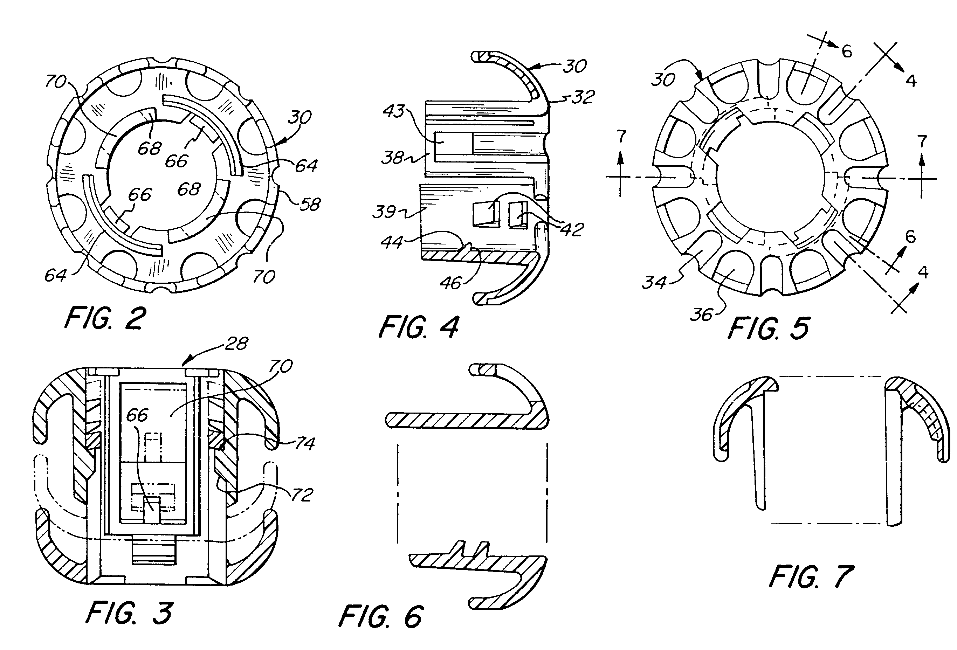

A first embodiment of an anastomotic device **28** is shown in Fig. 3 while a single unitary member, two of which make up device 28, is shown generally as 30 in **FIGS. 2, 4,** and **5**. Member 30 is made up of a ring member 32 having a plurality of slots 34 and apertures 36 about its periphery, a mating prong 38 which extends from ring member 32 in a direction substantially parallel to the center line of the ring member, and a separate prong 39 having engaging pawls formed therein. Mating prong 38 and separate prong 39 extending from the ring member 32 to form approximately one-half of the inner and outer portions of the ring member 32 as illustrated in **FIG. 5**.

Each mating prong 38 carries locking slots 43 designed and positioned to mate cooperatively with sets of engaging pawls 42 formed on the surface of separate prong 39. Each engaging pawl 42 is designed with a sloping forward edge 44 to facilitate easy relative motion when two unitary members 30 are joined and urged operatively together. Pawls 42 each have a vertical engaging edge 46 which functions with slots 43 to lock members 30 in place and form an anastomotic device.

A flatter ring member is desirable for use in an end to side anastomosis. The ring member that protrudes into the side of the tubular member is less obstructive to the flow path of the tubular member when it is flatter.

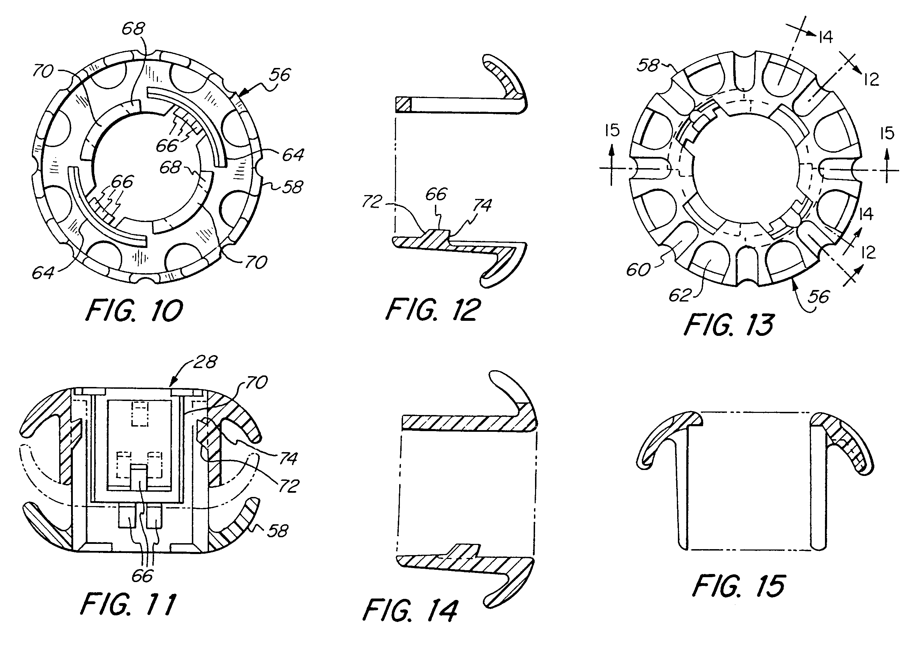

A second embodiment of anastomotic device with flatter ring members is shown in complete form in **FIG. 11** while a single unitary member, two of which make up this embodiment of device **28**, is generally as **56** in **FIGS. 10** and **13**. Member **56** is made up of a ring member **58** having a plurality of slots **60** and apertures **62** about its periphery and a pair of oppositely positioned depending legs **64**, each supporting a plurality of engaging pawls shown generally as **66**. Alternately positioned between depending legs **64** and opposite each other are depending members **68**, each of which has a pawl-engaging recess **70** to receive cooperatively pawls **66** when the two unitary members **56** are joined together to form anastomotic device **28**.

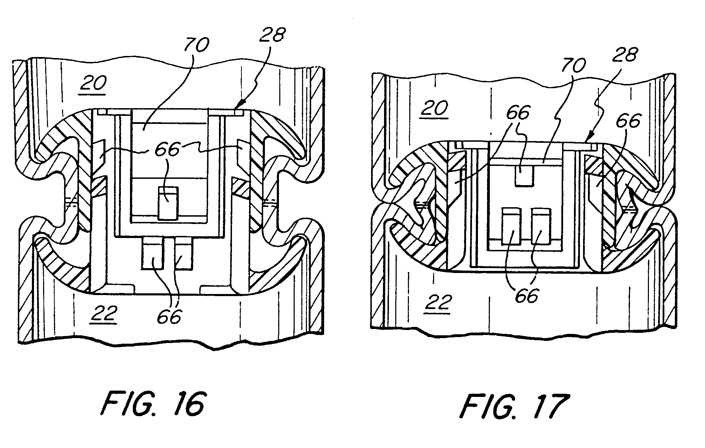

In the second embodiment of the anastomotic device, pawls **66** are selectively positioned on each depending leg **64** of unitary member **56** to enable the anastomotic device **28** formed from two members **56** to be positioned in a first fixed position as shown in solid lines in **FIG. 11** or in a second fixed position with the members closer together as shown in combined solid and broken lines in **FIG. 11**. The first and second fixed positions are also illustrated in **FIGS. 16** and **17** respectively. Obviously any number of pawls can be positioned on depending legs **66** to achieve other desired positions.

It is necessary that members **30** and **56** forming two embodiments of device **28** be made from a material that will permit disintegration of the device in a relatively short period of time once healing of the vessel ends commences. Acceptable materials for forming the device are disclosed in the prior art, and can be poly-hydroxyacetic ester and lactide copolymers. Molded surgical articles made from a wide range of glycolide/lactide copolymers have been known and utilized for quite some time.

The anastomotic device forming the present invention can be used in the same or a similar manner as anastomotic devices or staples disclosed in the prior art. Use of a purse-string suture permits engaging the very edge of the free end of the vessel wall so that suturing material can be pulled and the vessel end contracted much in the way the top of a purse or string-closed bag is manipulated. The ends 20 and 22 are then pulled over each ring member **32** so that the suture is tightened and the ends **20** and **22** of the vessel **24** and **26** are turned inwardly over ring members **32** as shown in **FIG. 8**. The members **32** are then urged together until the pawls **42** on the separate prongs **39** are engaged within the slots **43** on the mating prongs **38**, thus forming the connected annular coupling tube **40** of the device **28**. When members **32** are in the appropriate relationship by the formation of annular coupling tube **40**, the free ends of the two tubular members are contiguously positioned in a manner that will enable them to grow together permanently as shown in **FIG. 9**.

The device **28** provides ample space for the anastomosis to be performed after the members **32** are urged together and the mating prongs **38** engage with each other to form the annular coupling tube **40**. Coupling tube **40** is sturdy as a result of its formation by two contiguous rings of alternating mating and separate prongs best shown in **FIGS. 5** and **13**, Both members **30** and **56** have approximately one-half of an outer ring formed by the separate prong **39** in **FIG. 1** and approximately one-half of an inner ring formed by the mating prong **38**. Bowel tissue is mechanically held together by the force of the purse-string suture against the annular coupling tube **40** and the clamping force of the ring members compressing the two ends together.

Note that the edges **80** and **82** of member ends **20** and **22** are positioned to abut in an edge-to-edge relationship when the suture is tightened since the diameter of annular coupling tube **40** is not much smaller than the diameter of the ring **32**, thus resulting in a relatively unobstructed passageway through which fluids and gases may flow.

After member ends **20** and **22** are secured by purse string sutures, ring members **32** are then urged together until the pawls **42** on the separate prongs **39** are engaged within the slots **43** on the mating prongs **38**, thus forming the completed and connected annular coupling tube **40** as shown in **FIG. 9**. When ring members **32** are in the appropriate relationship and the formation of annular coupling tube **40** is complete, the ends of the members **20** and **22** are contiguously positioned at their edges **80** and **82** and at second adjacent locations **84** and **86** as shown in **FIG. 9**. Positioning the two tubular members in this manner will enable them to grow together permanently in the shortest amount of time.

A variety of diameters, spacings, and degrees of flatness for the ring members and diameters for the annular coupling tube are desirable to provide needed versatility of use within the needed sizes for animals and humans. Moreover, there are certain unusual tubular configurations within humans and animals that might better respond to the use of a similarly constructed anastomotic device having elliptically shaped ring members and annular coupling tubes rather than the circular configuration shown in the embodiment shown herein. Such embodiments are viewed as being within the scope of this disclosure and the accompanying claims.

It is to be understood that the anastomotic device made of either the materials referenced above or of other materials disclosed in the prior art can be designed to disintegrate, preferably through fragmentation in a given period of time. Such a material and design offers many advantages over conventional anastomotic devices and permits simple, rapid anastomosis in difficult areas. For example, it permits a simpler anastomosis in a low rectosigmoid anastomosis which ordinarily would be quite time-consuming and difficult, perhaps requiring temporary colostomy, or even being impossible in those cases necessitating a permanent colostomy.

It will be apparent that the present invention is comprised basically of two single unitary members, each having a disintegratable ring member and either a connected mating prong or depending legs and recess-forming members which cooperatively unite to form the embodiments described herein. However, the invention in its broader aspects is not limited to the specific embodiments herein shown and described, and departures may be made therefrom within the scope of the accompanying claim without departing from principles and without sacrificing its chief advantages.

## Claims

1. An anastomotic device for use in the surgical joining of the free ends of two tubular members to be anastomosed, said device characterized by a pair of ring members for securement to the free end of each of the tubular members to be anastomosed, the tubular member free ends each having terminal edges; annular connecting means joined to each of said ring members cooperatively mating with each other to connect said ring members and to form an interior passageway providing the sole flow path through the anastomotic device, the interior passageway being free from the presence of the free end terminal edges of the tubular members so that said free ends are joined outside the interior flow path of the anastomotic device and tubular members; and means, associated with said annular connecting means, for enabling the securement of said ring members at multiple predetermined distances from each other whereby said tubular free ends are ultimately positioned adjacent said ring members and said annular connecting means, and contiguous with each other when said ring members are secured at said multiple predetermined distances from each other; means extending around each tubular member free end holding said free end proximate said ring member and said annular connecting means, and contiguous to the other tubular member free end, and enabling said free ends to grow together in an atmosphere outside the flow path of the tubular members to be anastomosed and approximate the outer surface of the tubular member without substantial necrosis.

2. The device of claim 1 wherein said tubular member free ends are ultimately positioned over said ring members and around said annular connecting means.

3. The device of claim 1 or 2 wherein said extending means includes a purse-string suture, and said tubular member free end terminal edges are contiguous at said terminal edges and at a second location near said terminal edges.

4. The device of claim 3 wherein said ring members, said annular connecting means, and said purse-string suture are formed of a disintegratable material.

5. The device of claim 4, further comprising at least two mating prongs integral with said ring members and extending therefrom substantially parallel to the centerline of said ring members having lock slots formed therein, at least two separate prongs integral with said ring members and extending therefrom substantially parallel to the centerline of said ring members, said mating and separate prongs cooperatively connecting with each other to form an annular coupling tube.

6. The device of claim 4, further comprising at least two sets of prongs integral with said ring members and extending therefrom substantially parallel to the centerline of said ring members having lock slots formed therein, at least two sets of prongs integral with said ring members and extending therefrom substantially parallel to the centerline of said ring members, said mating sets of prongs cooperatively connecting with each other to form an annular coupling tube with an adjustable closed position.

7. The device of claim 4, further comprising said ring members flattened to lessen obstruction in the tubular member when an end to the side anastomosis of a tubular member is performed.

8. The device of claim 1 wherein said extending means includes a purse-string suture.

9. The device of claim 1 characterized by a pair of identical ring members for securement to the free end of each of the tubular members to be anastomosed, the tubular member free ends each having terminal edges; annular connecting means joined to each of said ring members cooperatively mating with each other to connect said ring members and to form an interior passageway providing the sole flow path through the anastomotic device, the interior passageway being free from the presence of the free end terminal edges of the tubular members so that said free ends are joined outside the interior flow path of the anastomotic device and tubular members; and means associated with said annular connecting means enabling the securement of said ring members at an adjustable distance from each other depending on the wall thickness of the tubular members, whereby said tubular member free ends are positioned contiguous of each other over said ring members and around said connecting means to enable the ends to grow together in an atmosphere outside the flow path of the tubular members to be anastomosed and approximate the outer surface of the tubular member.
